(19) Europäisches Patentamt
European Patent Office
Office européen des brevets

(11) **EP 0 856 181 B1**

(12) **EUROPÄISCHE PATENTSCHRIFT**

(45) Veröffentlichungstag und Bekanntmachung des Hinweises auf die Patenterteilung:
**10.03.1999 Patentblatt 1999/10**

(21) Anmeldenummer: **96945506.2**

(22) Anmeldetag: **17.10.1996**

(51) Int Cl.6: **G06F 19/00**
// **G06F159:00**

(86) Internationale Anmeldenummer:
**PCT/DE96/01975**

(87) Internationale Veröffentlichungsnummer:
**WO 97/15013 (24.04.1997 Gazette 1997/18)**

(54) **VERFAHREN UND VORRICHTUNG ZUR AUSWERTUNG EINES NARKOSE- ODER INTENSIV-EEG**

METHOD AND DEVICE FOR EVALUATING AN EEG CARRIED OUT IN THE CONTEXT OF ANAESTHESIA OR INTENSIVE CARE

PROCEDE ET DISPOSITIF POUR EVALUER UN EEG PRATIQUE DANS LE CADRE D'UNE ANESTHESIE OU DE SOINS INTENSIFS

(84) Benannte Vertragsstaaten:
**AT BE CH DE DK ES FI FR GB GR IE IT LI PT SE**

(30) Priorität: **19.10.1995 DE 19538925**

(43) Veröffentlichungstag der Anmeldung:
**05.08.1998 Patentblatt 1998/32**

(73) Patentinhaber: **Schultz, Arthur**
**29352 Adelheidsdorf (DE)**

(72) Erfinder:
• **SCHULTZ, Arthur**
**D-29352 Adelheidsdorf (DE)**
• **SCHULTZ, Barbara**
**D-29352 Adelheidsdorf (DE)**

(74) Vertreter: **Patentanwälte Thömen & Körner**
**Zeppelinstrasse 5**
**30175 Hannover (DE)**

(56) Entgegenhaltungen:
**WO-A-93/07804**     **US-A- 4 557 270**
**US-A- 5 083 571**

**Beschreibung**

Die Erfindung betrifft ein Verfahren zur Auswertung eines Narkose- oder Intensiv-EEG nach dem Oberbegriff des Anspruchs 1 und eine Vorrichtung zur Auswertung eines Narkose- oder Intensiv-EEG nach dem Oberbegriff des Anspruchs 5.

Die Elektroenzephalographie ist eine Methode zur Darstellung vom Gehirn erzeugter elektrischer Aktivität. In konventioneller Weise erfolgt die Registrierung des EEG mit einem Mehrkanalschreiber auf Endlospapier in der Regel mit einer Registriergeschwindigkeit von 30 mm/s. Die Deutsche EEG-Gesellschaft empfiehlt, Geräte mit mindestens zehn Kanälen zu verwenden. Um die Signale von den unterschiedlichen Regionen des Kopfes gut beurteilen zu können, werden die Elektroden in mehreren Ableiteprogrammen in unterschiedlicher Weise miteinander verschaltet. Zunehmend wird die Aufzeichnung auch mit Hilfe von Rechnern vorgenommen.

Die Zusammensetzung der Wellenformen im Elektroenzephalogramm (EEG) ist abhängig vom Funktionszustand des Gehirns. Die EEG-Bilder, die bei Patienten im Operations- und Intensivbereich auftreten, sind vielfältig und können durch eine große Zahl von endogenen und exogenen Faktoren beeinflußt werden. Neben dem normalen Wach-EEG ist z.B. mit Elementen des Schlaf-EEG, Effekten von Medikamenten und anderen exogen zugeführten chemischen Substanzen, ventilationsbedingten und metabolischen Einflüssen, Temperatureffekten, Folgen traumatischer Hirnläsionen sowie entzündlichen, vaskulären, degenerativen und durch Neoplasmen verursachten EEG-Veränderungen zu rechnen.

Folgenden Frequenzbereichen werden die im EEG auftretenden Wellen zugeordnet: Alpha (7.5 - 12.5 Hz), Beta (> 12.5 Hz), Theta (3.5 - 7.5 Hz) und Delta (0.5 - 3.5 Hz). Daneben können das Subdelta- (<0.5 Hz) und das Gamma-Band (>30 Hz) abgegrenzt werden. Bei der Befundung werden die Wellen in den Frequenzbereichen hinsichtlich ihrer Amplituden, Häufigkeit, Regelmäßigkeit, zeitlichen Gliederung, örtlichen Verteilung und Veränderung bei Reizen beschrieben. EEG-Amplituden werden in µV gemessen. Höherfrequente Wellen weisen in der Regel kleinere Amplituden auf, während mit einer Verlangsamung meist eine Amplitudenzunahme verbunden ist.

Zur Klassifikation von Schlaf-, Narkose- bzw. Koma-EEG-Stadien schlägt Kugler eine EEG-Einteilung vor, in der der Wachzustand mit A und EEG-Bilder bei fortschreitender Dämpfung der Hirnfunktion mit den Buchstaben B bis F bezeichnet werden. Zur Beurteilung der EEG-Kurven werden die Häufigkeit und Amplitude der Wellen in bestimmten Frequenzbereichen sowie typische Muster herangezogen.

Das Wach-EEG, Stadium A, ist bei der Mehrzahl der Erwachsenen durch Wellen im Alpha-Frequenzbereich gekennzeichnet. Stadium B ist charakterisiert durch Wellen mit schneller Frequenz und niedriger Amplitude. In den Stadien C bzw. D treten Theta- und Delta-Wellen auf. Im Stadium E bestimmt hochamplitudige Delta-Aktivität das Kurvenbild.

Stadium F ist durch einen Wechsel flacher bis isoelektrischer Kurvenstrecken und Gruppen höherer Wellen, das Burst-Suppression-Muster, oder durch eine kontinuierliche sehr flache Aktivität geprägt.

Die Ableitung eines konventionellen EEG ist relativ aufwendig. Die Interpretation erfordert spezielle Kenntnisse und Erfahrung. Eine bessere Beurteilung der dynamisch ablaufenden EEG-Veränderungen wird durch die Aufzeichnung des Original-Signales und der EEG-Spektralanalyse ermöglicht. Für eine Berechnung eines EEG-Leistungsspektrums werden für einen definierten Zeitabschnitt die EEG-Signale nach Analog-Digital-Wandlung einer Fast Fourier-Transformation (FFT) unterzogen. Mit Hilfe der Fourier-Transformation wird das Wellenbild des EEG in zugrundeliegende Schwingungskomponenten zerlegt, es erfolgt eine Umsetzung vom Zeit- in den Frequenzbereich. Die quadrierten Amplituden der Schwingungskomponenten bilden das Leistungs- oder Powerspektrum. Im EEG-Leistungsspektrum sind die im Zeitsignal auftretenden Frequenzen ablesbar. Aber auch diese Angaben bedürfen der Interpretation, um Aufschluß über das EEG-Stadium und damit über den cerebralen Funktionszustand zu erhalten.

Um eine automatische Mustererkennung zur erhalten, ist es aus der Zeitschrift "Biomedizinische Technik", 37, H. 6, 1992, S. 122 - 130 bekannt, autoregressive Parameter zur Klassifizierung von EEG-Abschnitten zu verwenden. Die Klassifizierung betrifft hier die Klassen A, C und E. Die erzielten Ergebnisse zeigen, daß autoregressive Parameter einen hohen Diskriminanzwert besitzen, und daß niedrige Klassifikationsfehlerraten bei Verwendung von quadratischen Diskriminanzfunktionen erreicht werden.

Der Erfindung liegt die Aufgabe zugrunde, ein Verfahren und eine Vorrichtung zur Auswertung eines Narkose-EEG oder Intensiv-EEG, anzugeben, bei dem aus den EEG-Kurven direkt alle Klassen des EEG-Stadiums eindeutig ermittelt werden können und Einflüsse durch Störungen weitest gehend vermieden werden.

Diese Aufgabe wird bei einem Verfahren nach dem Oberbegriff des Anspruch 1 durch die im Kennzeichen des Anspruchs 1 angegebenen Merkmale und bei einer Vorrichtung nach dem Oberbegriff des Anspruchs 6 durch die im Kennzeichen des Anspruchs 6 angegebenen Mermale gelöst.

EEG-Signale sind stochastische, d.h. zufallsabhängige, Prozesse, die sich mit Hilfe mathematisch-statistischer Verfahren näherungsweise beschreiben lassen. Für die rechnerische Weiterverarbeitung des EEG ist es aus praktischen Gründen wünschenswert, eine Reduktion auf wenige, möglichst aussagekräftige Parameter zu erzielen. Die Parameterbildung kann entweder im Zeit- oder im Frequenzbereich vorgenommen werden. Datengrundlage für Analysen im Zeitbereich ist das EEG-Signal, vor Berechnungen im Frequenzbereich erfolgt eine Transformation der Daten.

Eine Möglichkeit zur Analyse von EEG-Signalen im Frequenzbereich ist die Fourier-Analyse. Mit Hilfe der Fourier-Transformation wird das komplexe Wellenbild des EEG in die zugrundeliegenden Schwingungskomponenten zerlegt, es erfolgt eine Umsetzung vom Zeit- in den Frequenzbereich. Die quadrierten Amplituden dieser Schwingungskomponenten bilden das sogenannte Leistungs- oder Powerspektrum. Die Weiterverarbeitung der Ergebnisse der Fourier-Analyse umfaßt die Extraktion sogenannter Spektralparameter sowie weiterführende statistische Berechnungen. Zu den Parametern, die sich aus dem Spektrum ableiten lassen, gehören z.B. die Gesamtleistung sowie absolute und relative Leistungen in unterschiedlichen Frequenzbändern. Weitere häufig verwendete Parameter sind der Median, die Spectral Edge Frequency und die dominante Frequenz. Der Median ist die Frequenz, bei der die Fläche des Spektrums in zwei gleiche Teile geteilt wird. Die Spectral Edge Frequency wird meistens als 95 %-Quantil definiert, d.h. 95 % der Gesamtleistung des Spektrums liegen unterhalb dieser Frequenz. Die dominante Frequenz ist die Frequenz mit der größten Leistung.

Mit dem Leistungsspektrum läßt sich die Frequenzverteilung von EEG-Abschnitten übersichtlich darstellen. Auf spezielle Muster, wie Burst-Suppression-Phasen oder Anfallspotentiale, läßt sich aus dem Spektrum dagegen meist nicht schließen.

Ein Verfahren zur schnellen Berechnung des Leistungsspektrums ist die Fast Fourier-Transformation (FFT).

Eine Möglichkeit zur Analyse von EEG-Signalen im Zeitbereich ist die Ermittlung autoregressiver Parameter. Autoregressive (AR)-Parameter sind Größen aus dem Zeitbereich. Ein Meßwert zu einem bestimmten Zeitpunkt wird dargestellt als gewichtete Summe seiner Vergangenheitswerte plus einer Zufallskomponente. Die Gewichte sind die AR-Parameter. Die allgemeine Formel für einen AR-Prozeß lautet:

$$Y_t = a_1 * Y_{t-1} + ... + a_p * Y_{t-p} + e_t.$$

Hierbei bezeichnen $Y_t$ den Meßwert zum Zeitpunkt t, die $a_i$, i = 1 ,..., p die AR-Parameter und $e_t$ unabhängige Zufallskomponenten mit Mittelwert 0 und konstanter Varianz für alle Zeitpunkte t. Der Buchstabe p bezeichnet die Ordnung des Prozesses, d.h. die Anzahl der Vergangenheitswerte, die berücksichtigt werden. Die Modellparameter können mit Hilfe der Yule-Walker-Gleichungen geschätzt werden. Zur Ermittlung der Ordnung des Modells und der Überprüfung der Modellgüte wird üblicherweise der Ansatz von Box und Jenkins verwendet. Einen Überblick über weitere Schätzverfahren und Modellklassen geben Kay und Marple.

Eine häufig eingesetzte Methode zur Charakterisierung von EEG-Messungen ist die Berechnung spezieller EEG-Parameter, die von Hjorth vorgeschlagen und nach ihm benannt worden sind. Es handelt sich dabei um drei Parameter, und zwar Aktivität, Mobilität und Komplexität. Die Hjorth-Parameter werden aus der Streuung des EEG-Signals sowie deren erster und zweiter Ableitung berechnet. Alternativ kann die Berechnung der Hjorth-Parameter auch im Frequenzbereich, d.h. mit Hilfe der Spektralanalyse vorgenommen werden.

Die Aktivität entspricht der Gesamtleistung des Signals und ist damit ein Maß für die Amplitudengröße der EEG-Messung. Die Mobilität kann interpretiert werden als ein Maß für die mittlere Frequenz und die Komplexität als ein Maß für die Variabilität des Signals.

Neben reinen Spektralparametern oder reinen AR-Parametern ist die kombinierte Ermittlung von Spektralparametern, AR-Parametern, Hjorth-Parametern oder auch Chaosparametern möglich.

Für die Klassifikation von EEG-Daten mittels multivariater Klassifikationsfunktionen auf der Grundlage von Spektralparametern und/oder AR-Parametern und/oder Hjorth-Parametern und/oder Chaosparametern eignen sich z. B. diskriminanzanalytische Verfahren oder neuronale Netze.

Diskriminanzanalytische Klassifikationsverfahren sind dazu geeignet, Objekte anhand einer Reihe erhobener Merkmale einer von mehreren definierten Gruppen zuzuordnen. Bei der Narkose- oder Intensiv-EEG-Stadieneinteilung bilden die EEG-Abschnitte die zu klassifizierenden Objekte, die durch Spektralparameter und/oder AR- und/oder Hjorth-Parameter und/oder Chaosparameter charakterisiert sind. Zur Berechnung geeigneter Klassifikationsfunktionen existiert eine Reihe von Methoden, bei denen sich parametrische und nichtparametrische Ansätze unterscheiden lassen. Mittels einer Stichprobe von Objekten, für die die Gruppenzugehörigkeit bekannt ist, lassen sich Klassifikationsfunktionen basierend auf den betrachteten Merkmalswerten herleiten.

Bei parametrischen Verfahren wird vorausgesetzt, daß der betrachtete Merkmalsvektor in den verschiedenen Gruppen einer multivariaten Normalverteilung folgt. Die lineare Diskriminanzanalyse setzt die Gleichheit der Kovarianzmatrizen in den einzelnen Gruppen voraus, die quadratische Diskriminanzanalyse ermöglicht die Berücksichtigung unterschiedlicher Kovarianzmatrizen der Gruppen. Als Abstandsmaß wird die Mahalanobis-Distanz verwendet, die den gewichteten Abstand eines Beobachtungsvektors zu den Gruppenmittelwerten darstellt. Ein Objekt wird dann derjenigen Gruppe zugeordnet, bei der eine von dem gewählten Verfahren abhängige Funktion der Mahalanobis-Distanz am kleinsten ist.

Wenn die Verteilung des Merkmalsvektors unbekannt bzw. nicht normalverteilt ist, können nichtparametrische Verfahren zur Herleitung von Klassifikationsregeln eingesetzt werden. Ein anschauliches Verfahren ist die k-nearest-neighbor-Methode. Hierbei werden die Abstände des zu klassifizierenden Merkmalsvektors zu allen anderen Merkmalsvektoren der zur Verfügung stehenden Stichprobe gebildet, der Größe nach geordnet und die Beobachtungsvektoren mit den k kleinsten Abständen bestimmt, wobei die Anzahl k der berücksichtigten Werte vorher festgelegt werden muß. Dann wird bestimmt, zu welchen Gruppen diese k Werte gehören und deren Anteil an der Gesamtzahl der Messungen in den einzelnen Gruppen ermittelt. Die Zuordnung erfolgt dann zu der Gruppe, bei der dieser Anteil am größten ist.

Dieses nichtparametrische Verfahren erfordert einen erhöhten Rechenaufwand gegenüber parametrischen Methoden, da zur Klassifikation eines Objektes auf den gesamten Originaldatensatz zurückgegriffen werden muß, während bei parametrischen Methoden die Merkmalswerte eines Objektes in Klassifikationsfunktionen eingesetzt werden.

Zur Beurteilung der Güte eines Klassifikationsverfahrens kann die zugehörige Fehlerrate herangezogen werden, wobei unter Fehlerrate der Anteil von Fehlklassifikationen verstanden wird. Eine Möglichkeit zur Schätzung der Fehlerrate besteht in der Reklassifikation der Daten. Die so ermittelte Fehlerrate liefert jedoch eine zu positive Schätzung der wahren Fehlerrate. Eine realistischere Schätzung der Fehlerrate ist dann gegeben, wenn die Klassifikationen an einem unabhängigen Datensatz überprüft werden. Dies kann durch eine Aufsplittung des gegebenen Datensatzes in einen Trainingsdatensatz zur Herleitung der Klassifikationsvorschrift und einen Testdatensatz zur Validierung der Klassifikation erfolgen. Eine extreme Form des Aufsplittens der Daten besteht in der sogenannten Crossvalidation oder dem Leave-One-Out-Verfahren. Hierbei wird jeweils eine Beobachtung aus dem Datensatz herausgenommen und die Klassifikation anhand der aus den verbleibenden Daten berechneten Diskriminanzfunktion vorgenommen.

Liegt eine große Anzahl potentieller Merkmale zur Herleitung von Diskriminanzfunktionen vor, so lassen sich mit Hilfe geeigneter stufenweiser Verfahren diejenigen Parameter ermitteln, die eine größtmögliche Trennung der Gruppen gewährleisten. Zu diesem Zweck wird in der Literatur eine Reihe von Verfahren vorgeschlagen z. B. werden schrittweise Parameter in die Auswertung aufgenommen, die anhand von Wilks Lambda jeweils den größten Beitrag zur Gruppentrennung liefern.

Die Stadieneinteilung des Narkose- oder Intensiv-EEG kann in Anlehnung an Kugler erfolgen, der, wie einleitend erwähnt wurde, den Wachzustand mit A und die sehr tiefe Dämpfung der Hirnfunktion mit F bezeichnet. Die Zwischenstadien B bis E können dabei noch weiter unterteilt werden, wie Tabelle 1 zeigt.

Tabelle 1

| STADIUM | FÜHRENDE EEG-KENNZEICHEN | VERHALTEN |
|---|---|---|
| Ao | Alpha okz. Normvariante | Wachheit |
| $A_1$ | Alpha -Diffusion | Subvigilanz |
| $A_2$ | Alpha niedrig, spärlich, langsam Theta niedrig | |
| $B_0$ | Theta niedrig | Schläfrigkeit |
| $B_1$ | Alpha vereinzelt | bzw. |
| $B_2$ | Theta niedrig bis mittelhoch Theta mittelhoch | sehr flache Narkose |
| $C_0$ | Theta hoch 30% der Zeit | leichter Schlaf |
| $C_1$ | Theta hoch 50% der Zeit | bzw. |
| $C_2$ | Theta hoch, langsam, kontinuierlich | flache Narkose |
| $D_0$ | Delta bis 30% der Zeit | mittlerer Schlaf |
| $D_1$ | Delta bis 50% der Zeit | bzw. |
| $D_2$ | Delta bis 80% der Zeit | mittlere Narkose |
| E | Delta kontinuierlich | tiefer Schlaf bzw. tiefe Narkose |
| F | periodisch langsame Gruppen/flache Strecken | Koma bzw. sehr tiefe Narkose |

Es können auch andere Stadieneinteilungen bzw. -skalen verwendet werden.

Mit dem beschriebenen Verfahren und der Vorrichtung ist es möglich, diese bisher visuell anhand der EEG-Kurven vorgenommene Stadieneinteilung des Narkose- oder Intensiv-EEG rechnergesteuert automatisch vorzunehmen. Dies hat den Vorteil, daß eine einheitliche Stadieneinteilung erfolgt, die von Fehlern durch unterschiedliche individuelle Beurteilung frei ist. Das Verfahren und die Vorrichtung eignen sich gleichermaßen für die Überwachung von Narkose- und Intensivpatienten im klinischen Bereich wie auch in der pharmazeutischen Industrie bei der Entwicklung und Erprobung sowie der Ermittlung von Dosierangaben neuer Narkotika und Sedativa.

Während mit den bisherigen Maßnahmen nur die Stadien A bis E sicher erkannt werden, ermöglicht die Ermittlung zusätzlicher Grenzwerte der Parameter, insbesondere der Signalleistung bezogen auf eine Null-Linie, und eine Korrektur der multivariaten Klassifikation anhand der Grenzwerte eine wesentliche Verbesserung der Erkennbarkeit des Stadiums F der Stadieneinteilung. Da dieses Stadium im Gegensatz zu den anderen Stadien teilweise durch eine Null-Linie charakterisiert sein kann, führt die ausschließliche Auswertung von Spektral-, AR-, Hjorth- oder Chaosparametern Parametern nicht immer zu einer sicheren Erkennung des Stadiums F.

Da die vom Gehirn erzeugte elektrische Aktivität ein sehr geringes elektrisches Potential darstellt, ist ein EEG extrem störanfällig. Im wesentlichen werden EEG-Kurven durch Bewegungsartefakte, Muskelartefakte oder Störstrahlung beeinflußt. Diese Störungen können einerseits die Auswertung eines EEG zeitweise völlig unmöglich machen, andererseits aber auch elektrische Aktivität vortäuschen, die nicht vom Gehirn stammt und somit zu Fehlinterpretationen führen würde.

Wenn Bewegungsartefakte z. B. bei einer Operation in Anwesenheit medizinischen Personals auftreten, so können falsche Stadieneinteilungen natürlich ignoriert werden, wenn die Bewegung wahrgenommen wird. Bei einer Aufzeichnung des EEG und späteren Auswertung ist es aber nicht mehr möglich, aus der EEG-Kurve festzustellen, ob bestimmte Signale durch Bewegungsartefakte hervorgerufen wurden oder nicht.

Indem unabhängig von der EEG-Aufzeichnung zusätzlich Bewegungsartefakte ermittelt werden, kann anhand der Bewegungsartefakte die Stadieneinteilung korrigiert oder unterdrückt werden.

Eine weitere Verbesserung der Stadieneinteilung wird erreicht, wenn aus gespeicherten unterschiedlichen altersabhängigen Klassifikationsfunktionen die für einen Probanden altersspezifischen Klassifikationsfunktionen ausgewählt werden. Es wurde nämlich herausgefunden, daß das EEG eines Menschen altersabhängige Charakteristika aufweist. Vereinfacht ausgedrückt verschiebt sich z.B. das Spektrum bei Erwachsenen mit zunehmendem Alter im Wachzustand zu niedrigeren Frequenzen, während der Narkose ist z.B. die Delta-Leistung vermindert. Durch Berücksichtung altersspezifischer Klassifikationsfunktionen kann die richtige Stadieneinteilung zuverlässiger getroffen werden.

Weiterhin können auch aus gespeicherten unterschiedlichen medikamentenabhängigen Klassifikationsfunktionen die für ein eingesetztes Medikament spezifischen Klassifikationsfunktionen ausgewählt werden. Es hat sich gezeigt, daß unterschiedliche schlafinduzierende Medikamente, also Narkotika oder Sedativa, in einzelnen Stadien zu unterschiedlichen EEG-Kurven führen. Bei pauschal gewählten Klassifikationsfunktionen könnte dies zu Fehlinterpretationen und damit zu einer fehlerhaften Zuordnung der Stadieneinteilung des Narkose- oder Intensiv-EEG führen. Durch Berücksichtigung spezifischer Klassifikationsfunktionen für ein eingesetztes Medikament können solche falschen Zuordnungen vermieden werden.

Zur Ermittlung von Bewegungsartefakten kann ein mit einem zur Auswertung dienenden Rechner gekoppelter Bewegungssensor mit wenigstens einem am Kopf des Patienten oder Probanden anbringbaren Sensorelement vorgesehen sein. Als Sensorelemente sind Beschleunigungsaufnehmer und/oder Verformungsaufnehmer besonders geeignet.

Die Sensorelemente können aber auch als mit dem Kopf des Patienten oder Probanden gekoppelte elektrische Leitungen ausgebildet sein, die sich in einem Magnetfeld und/oder elektrischem Feld befinden. Bei Bewegungen der Leitungen im Feld und/oder Änderungen der Feldstärke unter dem Einfluß benachbarter bewegter Körper oder Gegenstände werden dann in den Leitungen Ströme induziert und/oder Spannungen erzeugt, die sich auswerten lassen.

Zusätzlich können auch Muskelartefakte aus dem Zeit- und/oder Frequenzbereich der EEG-Kurven ermittelt werden und anhand der Muskelartefakte die Stadieneinteilung korrigiert oder unterdrückt werden. Solche Muskelartefakte können durch die Stirn- und Gesichtsmuskulatur hervorgerufen werden und sind meist daran erkennbar, daß neben anderen Frequenzanteilen auch Frequenzanteile größer 30 Hz vorhanden sind.

Schließlich können auch zusätzlich Artefakte durch Störstrahlung elektromedizinischer Geräte, insbesondere elektrochirurgischer Geräte ermittelt werden und anhand der Störstrahlungsartefakte die Stadieneinteilung der Narkosetiefe korrigiert oder unterdrückt werden. In diesem Fall ist ein mit dem Rechner gekoppelter Störstrahlungssensor vorgesehen, der die Störstrahlung erfaßt.

Artefakte lassen sich aber auch durch Softwareabfragen erkennen.

Nachfolgend wird die Erfindung anhand eines Ausführungsbeispiels erläutert, das in der Zeichnung dargestellt ist. In der Zeichnung zeigen:

Fig. 1       eine Vorrichtung aus einem Personal Computer und einer Meßeinheit für zwei EEG-Kanäle.

Fig. 2       eine Bildschirmdarstellung und

Fig. 3       grafische Darstellungen zweier EEG-Abschnitte.

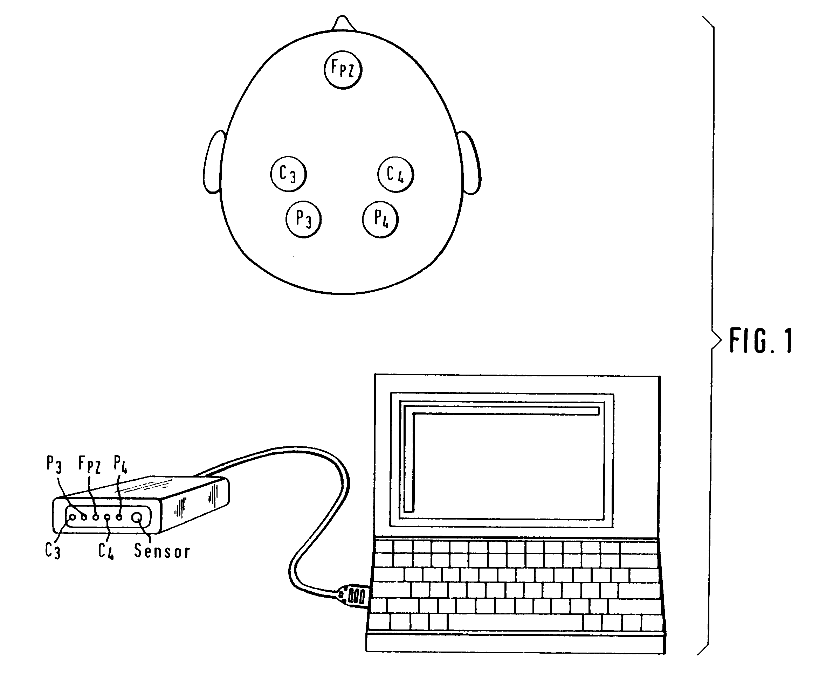

Die in Fig. 1 dargestellte Vorrichtung besteht aus einem Personal Computer mit Farbbildschirm und einer Meßeinheit für zwei EEG-Kanäle. Bei EEG-Registrierungen werden Elektroden auf den Kopf des Patienten geklebt und an den in unmittelbarer Nähe des Patienten plazierten Vorverstärker angeschlossen. In einem Differenzverstärker erfolgt die Bildung der Differenz zwischen den Potentialen zweier Elektroden. Die Signale werden um den Faktor 20.000 verstärkt, um eine Wertebereichsanpassung an den AD(Analog/Digital)-Wandler vorzunehmen.

Zur Erkennung von Bewegungsartefakten ist ein Bewegungssensor am Kopf des Patienten befestigt. Dessen Signale werden ebenfalls einer AD-Wandlung unterzogen.

Vor der AD-Wandlung wird eine Hoch- und Tiefpaß- sowie Notch-Filterung vorgenommen. Ein 50-Hz-Notch-Filter wirkt Netzbrumm-Artefakten entgegen. Die gefilterten und verstärkten Analogsignale werden mit 128 Hz digitalisiert. Dies wird von einem Microcontroller gesteuert und dieser stellt dem PC die Werte zur Weiterverarbeitung zur Verfügung. Dieser übernimmt die Speicherung und Auswertung der Daten. Die EEG-Analyse erfolgt parallel zur AD-Wandlung, wobei die Kontinuität der Datenaufnahme gesichert ist.

Nach Einschalten des Gerätes erscheint auf dem Bildschirm ein Auswahl-Menü, in dem gewählt werden kann, ob das Meßprogramm gestartet oder eine schon gespeicherte Messung eingelesen werden soll.

Die Programmbedienung erfolgt wahlweise über eine integrierte Folientastatur, einen Touch-Screen oder über eine externe Tastatur. Ausgaben werden über den Bildschirm und optional über einen Drucker vorgenommen.

Vor jeder Messung wird das Patientenalter eingegeben. Wahlweise können die Daten von einer Ableitung oder von zwei Ableitungen ausgewertet und gespeichert werden.

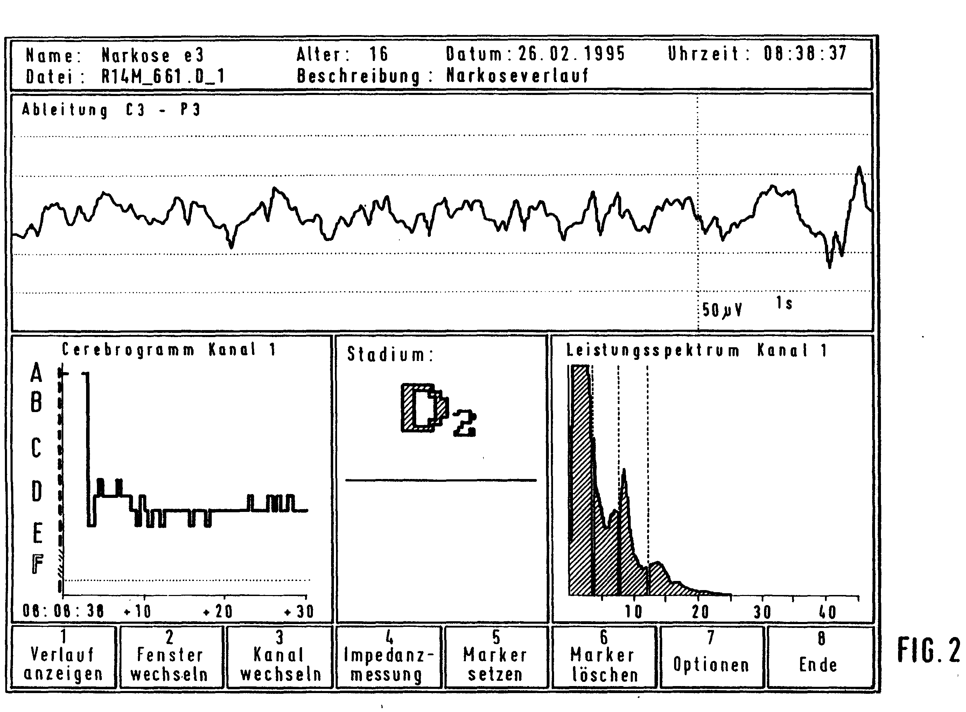

Mit Beginn der Messungen wird auf dem Bildschirm gemäß Fig. 2 fortlaufend das Original-EEG von einer oder zwei Ableitungen, ein aus zehn Zwei-Sekunden-Epochen gemitteltes EEG-Leistungsspektrum mit Kennzeichnung der Grenzen der konventionellen EEG-Frequenzbänder, das aktuelle Stadium (von A bis F) und der Stadienverlauf als Cerebrogramm dargestellt.

Zusätzlich werden der Dateiname, das Patientenalter, Datum und Uhrzeit sowie optional Kommentare ausgegeben.

Statt des Leistungsspektrums lassen sich auch Trends von Quantilen des Leistungsspektrums sowie von den relativen Leistungen des Alpha-, Beta-, Theta- und Deltabandes in kumulativer Form darstellen.

Die Stadienklassifikation wird anhand diskriminanzanalytischer Funktionen sowie ergänzender Grenzwertabfragen vorgenommen. Zusätzlich wurden Algorithmen zur Verbesserung der Burst-Suppression-Phasen-Erkennung (Stadium F) programmiert. Zwei-Sekunden-Epochen, die als artefaktbelastet betrachtet werden, finden bei der Stadienklassifikation keine Berücksichtigung.

Bei der Aufstellung diskriminanzanalytischer Funktionen wird von k Stadien sowie einem p-dimensionalen Merkmalsvektor $X = (X_1,..X_p)$ (z.B. p geeignete EEG-Parameter) ausgegangen. Basierend auf dem Merkmalsvektor X wird eine Zuordnung zu einem der k Stadien vorgenommen.

Die Herleitung von Klassifikationsfunktionen, mit denen die Zuordung vorgenommen wird, erfolgt anhand eines Trainingsdatensatzes. Der Trainingsdatensatz enthält EEG-Signale, die für die unterschiedlichen Stadien typisch sind. Hieraus werden mit Hilfe diskriminanzanalytischer Verfahren Klassifikationsfunktionen für die k Stadien berechnet.

Im Klassifikationsfall werden aus dem betrachteten EEG-Abschnitt Parameter (z.B. Spektral-, AR-, Hjorth- oder Chaosparameter) berechnet und in die aus dem Trainingsdatensatz ermittelten Klassifikationsfunktionen eingesetzt. Die Klassifikationsfunktionen liefern eine Zuordnungswahrscheinlichkeit für jedes der k Stadien, d.h. ein Maß für die Ähnlichkeit des betrachteten EEG-Abschnitts zu den verschiedenen Stadien. Der EEG-Abschnitt wird dann dem Stadium mit der größten Zuordnungswahrscheinlichkeit zugeordnet.

## Beispiel

Anhand eines konkreten Beispiels soll nun die Vorgehensweise bei der Diskriminanzanalyse veranschaulicht werden.

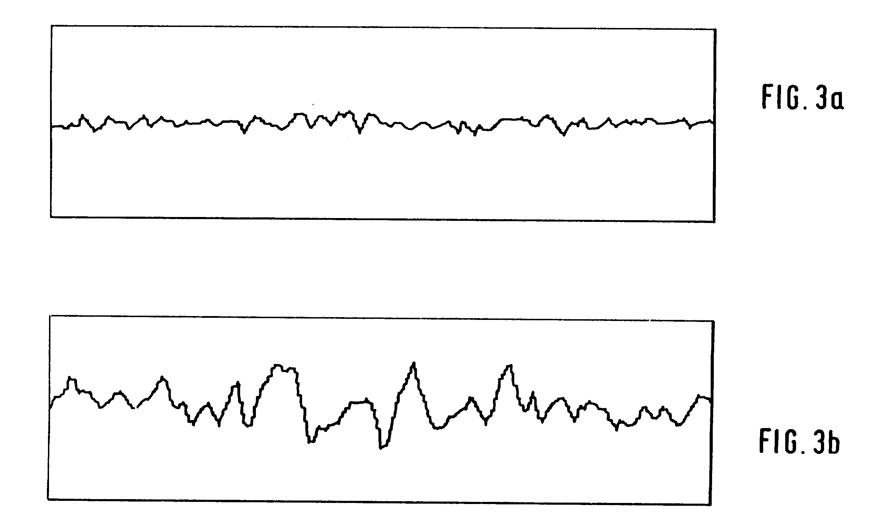

Gegeben sind die in Fig. 3 dargestellten EEG-Abschnitte von jeweils 2 Sekunden Dauer, die mit Hilfe der Diskriminanzanalyse einem Stadium von A (wach) bis E (tiefe Narkose) zugeordnet werden sollen. Die Stadieneinteilung erfolgte in Anlehnung an Kugler. Bei Abschnitt (a) handelt es sich um einen EEG-Abschnitt aus flacher Narkose, bei Abschnitt (b) um ein EEG aus tieferer Narkose.

Der erste Schritt besteht in der Berechnung geeigneter Parameter zur Charakterisierung des jeweiligen EEG-Abschnitts. In diesem Fall wurden fünf autoregressive Parameter sowie die logarithmierte Standardabweichung, die der Leistung des EEG-Signals entspricht, berechnet. Es können aber auch andere, z.B. Spektral- oder Hjorth-Parameter, verwendet werden. Ein EEG-Abschnitt wird also beschrieben durch den Merkmalsvektor X, der die oben genannten sechs EEG-Parameter als Komponenten enthält.

Anhand des Trainingsdatensatzes, bei dem die Stadienzugehörigkeit aufgrund einer visuellen Auswertung bekannt ist, werden dann die Diskriminanzfunktionen berechnet. Im vorliegenden Fall wurde die lineare Diskriminanzanalyse für 12 Stadien A, $B_0$, $B_1$, $B_2$, $C_0$, $C_1$, $C_2$, $D_0$, $D_1$, $D_2$, $E_0$ und $E_1$ verwendet.

Zur konkreten Klassifikation eines neu zu klassifizierenden EEG-Abschnitts wird zunächst der zugehörige Merkmalsvektor X berechnet. Für die vorliegenden zwei EEG-Abschnitte (a) und (b) ergeben sich folgende Werte

(a) X = (1.20, 1.45, -0.89, 0.14, 0.06, 0.03),
(b) X = (2.32, 1.69, -0.96, 0.18, 0.05, -0.06),

wobei die erste Komponente die logarithmierte Standardabweichung des Signals bezeichnet und die weiteren Komponenten die fünf autoregressiven Parameter darstellen.

Diese Werte werden dann in die vorhandenen Klassifikationsfunktionen eingesetzt, und man erhält für jedes der möglichen Stadien die zugehörige Zuordnungswahrscheinlichkeit.

Die errechneten Werte für die EEG-Abschnitte (a) und (b) sind in Tabelle 2 dargestellt. Die Klassifikation erfolgt zu dem Stadium, dem der EEG-Abschnitt am ähnlichsten ist, d.h. zu dem Stadium mit der größten Zuordnungswahrscheinlichkeit. Tabelle 2 entnimmt man, daß EEG-Abschnitt (a) dem Stadium $B_1$ und Abschnitt (b) dem Stadium $D_0$ zugeordnet wird.

Tabelle 2

| Zuordnungswahrscheinlichkeiten für die EEG-Abschnitte aus Fig. 3 | | | | | | | | | | | | |
|---|---|---|---|---|---|---|---|---|---|---|---|---|
| | Zuordnungswahrscheinlichkeiten für die Stadien | | | | | | | | | | | |
| Abschnitt | A | $B_0$ | $B_1$ | $B_2$ | $C_0$ | $C_1$ | $C_2$ | $D_0$ | $D_1$ | $D_2$ | $E_0$ | $E_1$ |
| (a) | 0.00 | 0.25 | 0.69 | 0.04 | 0.02 | 0.00 | 0.00 | 0.00 | 0.00 | 0.00 | 0.00 | 0.00 |
| (b) | 0.00 | 0.00 | 0.00 | 0.00 | 0.01 | 0.11 | 0.31 | 0.38 | 0.19 | 0.00 | 0.00 | 0.00 |

## Patentansprüche

1. Verfahren zur Auswertung eines Narkose- oder Intensiv-EEG, bei dem aus den EEG-Kurven Parameter aus dem Zeit- und/oder Frequenzbereich ermittelt werden, indem die ermittelten Parameter in multivariate Klassifikationsfunktionen eingesetzt werden und daraus automatisch eine Stadieneinteilung des Narkose- oder Intensiv-EEG vorgenommen wird, dadurch gekennzeichnet, daß zusätzlich Grenzwerte der Parameter, insbesondere der Signalleistung bezogen auf eine Null-Linie ermittelt werden und anhand der Grenzwerte eine Korrektur der multivariaten Klassifikation vorgenommen wird und daß Bewegungsartefakte ermittelt werden und anhand der Bewegungsartefakte die Stadieneinteilung des Narkose- oder Intensiv-EEG korrigiert oder unterdrückt wird.

2. Verfahren nach Anspruch 1, dadurch gekennzeichnet, daß aus gespeicherten unterschiedlichen altersabhängigen Klassifikationsfunktionen die für einen Probanden altersspezifischen Klassifikationsfunktionen ausgewählt werden.

3. Verfahren nach Anspruch 1 oder 2, dadurch gekennzeichnet, daß aus gespeicherten unterschiedlichen medikamentenabhängigen Klassifikationsfunktionen die für ein eingesetztes Medikament spezifischen Klassifikationsfunktionen ausgewählt werden.

4. Verfahren nach einem der Ansprüche 1 bis 3, dadurch gekennzeichnet, daß zusätzlich Muskelartefakte aus dem Zeit- und/oder Frequenzbereich der EEG-Kurven ermittelt werden und anhand der Muskelartefakte die Stadieneinteilung des Narkose- oder Intensiv-EEG korrigiert oder unterdrückt wird.

5. Verfahren nach einem der Ansprüche 1 bis 4, dadurch gekennzeichnet, daß zusätzlich Artefakte durch Störstrahlung elektromedizinischer Geräte, insbesondere elektro-chirurgischer Geräte ermittelt werden und anhand der Störstrahlungsartefakte die Stadieneinteilung des Narkose- oder Intensiv-EEG korrigiert oder unterdrückt wird.

6. Vorrichtung zur Auswertung eines Narkose- oder Intensiv-EEG mittels eines Rechners, der aus den gemessenen EEG-Kurven Parameter aus dem Zeit- und/oder Frequenzbereich ermittelt, die ermittelten Parameter in multivariate Klassifikationsfunktionen einsetzt und daraus automatisch eine Stadieneinteilung des Narkose- oder Intensiv-EEG vornimmt, <u>dadurch gekennzeichnet,</u> daß der Rechner zusätzlich Grenzwerte der Parameter, insbesondere der Signalleistung bezogen auf eine Null-Linie ermittelt und anhand der Grenzwerte eine Korrektur der multivariaten Klassifikation vornimmt, und daß ein mit dem Rechner gekoppelter Bewegungssensor mit wenigstens einem am Kopf des Patienten oder Probanden anbringbaren oder mit diesem koppelbaren Sensorelement vorgesehen ist und daß vom Bewegungssensor ermittelte Bewegungsartefakte vom Rechner in der Weise auswertbar sind, daß die Stadieneinteilung des Narkose- oder Intensiv-EEG korrigiert oder unterdrückt wird.

7. Vorrichtung nach Anspruch 6, <u>dadurch gekennzeichnet,</u> daß in einem Speicher des Rechners unterschiedliche altersabhängige Klassifikationsfunktionen gespeichert sind, und daß aus den gespeicherten Klassifikationsfunktionen durch Eingabe einer Altersangabe altersspezifische Klassifikationsfunktionen auswählbar sind.

8. Vorrichtung nach Anspruch 6 oder 7, <u>dadurch gekennzeichnet,</u> daß in einem Speicher des Rechners zusätzlich unterschiedliche medikamentenabhängige Klassifikationsfunktionen gespeichert sind, und daß aus den gespeicherten Klassifikationsfunktionen durch Eingabe einer Medikamentenangabe die für ein eingesetztes Medikament spezifischen Klassifikationsfunktionen auswählbar sind.

9. Vorrichtung nach einem der Ansprüche 6 bis 8, <u>dadurch gekennzeichnet,</u> daß die Sensorelemente Beschleunigungsaufnehmer und/oder Verformungsaufnehmer sind.

10. Vorrichtung nach einem der Ansprüche 6 bis 8, <u>dadurch gekennzeichnet,</u> daß die Sensorelemente als mit dem Kopf des Patienten oder Probanden gekoppelte elektrische Leitungen ausgebildet sind, die sich in einem Magnetfeld und/oder elektrischen Feld befinden und bei Bewegungen im Feld und/oder Änderungen der Feldstärke auswertbare elektrische Ströme und/oder elektrische Spannungen erzeugen.

11. Vorrichtung nach einem der Ansprüche 6 bis 10, <u>dadurch gekennzeichnet,</u> daß durch den Rechner Muskelartefakte aus dem Zeit- und/oder Frequenzbereich der EEG-Kurven in der Weise auswertbar sind, daß die Stadieneinteilung des Narkose- oder Intensiv-EEG korrigiert oder unterdrückt wird.

12. Vorrichtung nach einem der Ansprüche 6 bis 11, <u>dadurch gekennzeichnet,</u> daß ein mit dem Rechner gekoppelter Störstrahlungssensor vorgesehen ist, und daß vom Störstrahlungssensor ermittelte Störstrahlung elektromedizinischer Geräte, insbesondere elektrochirurgische Geräte, vom Rechner in der Weise auswertbar sind, daß die Stadieneinteilung des Narkose- oder Intensiv-EEG korrigiert oder unterdrückt wird.

**Claims**

1. Method for evaluating a narcotic or intensive EEG, in which parameters of the time and/or frequency range are determined from the EEG curves, whereby the determined parameters are employed in multivariate classification functions and a stage classification of the narcosis or intensive EEG is undertaken automatically on this basis, <u>characterised in that,</u> in addition, limiting values of the parameters, in particular of the signal output with reference to zero, are determined and a correction of the multivariate classification made on the basis of the limiting values, and that motion artefacts are determined and the stage classification of the narcotic or intensive EEG is corrected or suppressed on the basis of the motion artefacts.

2. Method according to Claim 1, <u>characterised in that</u> age-specific classification functions are selected for a test person from different, stored, age-dependent classification functions.

3. Method according to Claim 1 or 2, <u>characterised in that</u> classification functions specific to a medication used are selected from different, stored, medication-dependent classification functions.

4. Method according to one of Claims 1 to 3, <u>characterised in that,</u> in addition, muscle artefacts are determined from the time and/or frequency range of the EEG curves and the stage classification of the narcotic or intensive EEG is corrected or suppressed on the basis of the muscle artefacts.

5. Method according to one of Claims 1 to 4, <u>characterised in that,</u> in addition, artefacts resulting from interfering radiation from electromedical devices, in particular electrosurgical devices, are determined and the stage classification of the narcotic or intensive EEG is corrected or suppressed on the basis of the interfering radiation artefacts.

6. Device for evaluating a narcotic or intensive EEG using a computer, which determines parameters of the time and/ or frequency range from the measured EEG curves, uses the determined parameters in multivariate classification functions, and automatically carries out a stage classification of the narcotic or intensive EEG on this basis, characterised in that the computer also determines limiting values of the parameters , in particular of the signal output with reference to zero, and uses the limiting values to undertake a correction of the multivariate classification, and in that a motion sensor connected to the computer is equipped with at least one sensor element which can be fitted to or connected to the head of the patient or test person, and in that motion artefacts determined by the motion sensor can be evaluated by the computer in such a way that the stage classification of the narcotic or intensive EEG is corrected or suppressed.

7. Device according to Claim 6, characterised in that different age-dependent classification functions are stored in the computer memory, and that inputting an age allows age-specific classification functions to be selected from the stored classification functions.

8. Device according to Claim 6 or 7, characterised in that, in addition, different medication-dependent classification functions are stored in the computer memory, and that inputting medication details allows the classification functions specific to the medication used to be selected from the stored classification functions.

9. Device according to one of Claims 6 to 8, characterised in that the sensor elements are acceleration sensors and/ or deformation sensors.

10. Device according to one of Claims 6 to 8, characterised in that the sensor elements are implemented as electrical leads which are connected to the head of the patient or test person, these leads being located in a magnetic and/ or electrical field and, in the event of motion in the field and/or changes in field intensity, generating electrical currents and/or electrical voltages which can be evaluated.

11. Device according to one of Claims 6 to 10, characterised in that muscle artefacts from the time and/or frequency range of the EEG curves can be evaluated by the computer in such a way that the stage classification of the narcotic or intensive EEG is corrected or suppressed.

12. Device according to one of Claims 6 to 11, characterised in that an interfering radiation sensor, connected to the computer, is provided, and that interfering radiation from electromedical devices, in particular electrosurgical devices, determined by the interfering radiation sensor can be evaluated by the computer in such a way that the stage classification of the narcotic or intensive EEG is corrected or suppressed.

**Revendications**

1. Procédé d'évaluation d'un EEG pratique dans le cadre d'une anesthésie ou de soins intensifs, dans lequel, sur la base des courbes de EEG, des paramètres appartenant aux domaines du temps et/ou des fréquences sont déterminés, en liant les paramètres déterminés à des fonctions de classification multivariables et en procédant, à partir de cela, automatiquement à une division en stades de EEG dans le cadre d'anesthésie ou de soins intensifs, caractérisé en ce que des valeurs limites additionnelles des paramètres, en particulier de la puissance de signal par rapport à une ligne zéro, sont déterminées, et qu'à l'aide des valeurs limites, une correction de la classification multivariable est effectuée, et en ce que des artefacts de mouvement sont détectés et qu'à l'aide des artefacts de mouvement, la division en stades de EEG pratique dans le cadre d'une anesthésie ou de soins intensifs est corrigée ou supprimée .

2. Procédé selon la revendication 1, caractérisé en ce que, parmi différentes fonctions mémorisées suivant l'âge, sont choisies les fonctions de classification spécifiques à l'age pour un propositus.

3. Procédé selon la revendication 1 ou 2, caractérisé en ce que, parmi différentes fonctions mémorisées dépendant du médicament, sont choisies les fonctions de classification spécifiques pour un médicament utilisé.

4. Procédé selon une des revendications 1 à 3, caractérisé en ce que des artefacts additionnels de muscles sont déterminés à partir du domaine du temps et des fréquences des courbes d'EEG et qu' à l'aide des artefacts de muscles, est corrigée ou supprimée la division en stades d'EEG pratique dans le cadre d'une anesthésie ou de soins intensifs.

5. Procédé selon une des revendications 1 à 4, caractérisé en ce que on détermine des artefacts additionnels dus au rayonnement perturbateur d'appareils électromédicaux, en particulier d'appareils électrochirurgicaux, et qu' à l'aide des artefacts de rayonnement perturbateur, est corrigée ou supprimée la division en stades d'EEG pratique dans le cadre d'une anesthésie ou de soins intensifs.

6. Dispositif d'évaluation d'une EEG pratique d'anesthésie ou de soins intensifs au moyen d'un calculateur qui détermine des paramètres appartenant au domaine du temps ou des fréquences sur la base des courbes d'EEG mesurées, qui lie les paramètres obtenus à des fonctions de classification multivariables et qui procède, à partir de là, automatiquement à une division en stades d'EEG pratique dans le cadre d'une anesthésie ou de soins intensifs, caractérisé en ce que le calculateur détermine des valeurs limites additionnelles de ces paramètres, en particulier de la puissance de signal par rapport à une ligne zéro, qu'il procède, à l'aide des valeurs limites, à une correction de la classification multivariable et, en ce qu'un détecteur de mouvement couplé au calculateur est pourvu d'un élément détecteur au moins pouvant être fixé sur la tête du patient ou du propositus, ou couplé à celle-ci, et que des artefacts de mouvement détectés par le détecteur de mouvement peuvent être évalués par le calculateur de manière que soit corrigée ou supprimée la division en stades d'EEG pratique dans le cadre d'une anesthésie ou de soins intensifs.

7. Dispositif selon la revendication 6, caractérisé en ce que des fonctions de classification différentes dépendantes de l'âge sont mémorisées dans la mémoire du calculateur, et que des fonctions de classification spécifiques à l'âge peuvent être choisies parmi les fonctions de classification mémorisées en entrant l'indication d'un age.

8. Dispositif selon une des revendications 6 ou 7, caractérisé en ce que, dans une mémoire du calculateur sont mémorisées, en plus, des fonctions de classification différentes dépendantes de médicaments, et que des fonctions de classification spécifiques pour un médicament utilisé peuvent être choisies en entrant un nom de médicament.

9. Dispositif selon une des revendications 6 à 8, caractérisé en ce que les éléments détecteurs sont des capteurs d'accélération et/ou des capteurs de déformation.

10. Dispositif selon une des revendications 6 à 8, caractérisé en ce que les éléments détecteurs sont conçus comme conducteurs électriques qui sont couplés à la tête du patient ou du propositus, qu'ils se trouvent dans un champ magnétique et/ou électrique et qu'ils produisent des courants et/ou des tensions électriques pouvant être évalués, lorsqu'il y a des mouvements dans le champ et/ou des modifications d'intensité du champ.

11. Dispositif selon une des revendications 6 à 10, caractérisé en ce que le calculateur peut évaluer des artefacts de muscles à partir du domaine du temps et/ou des fréquences des courbes d'encéphalographie, de manière que la division en stades d'EEG pratique dans le cadre d'une anesthésie ou de soins intensifs intensive soit corrigée ou supprimée.

12. Dispositif selon une des revendications 6 à 11, caractérisé en ce qu' un détecteur de rayonnement perturbateur est prévu, qui est couplé au calculateur et que le rayonnement perturbateur d'appareils électromédicaux, en particulier, électrochirurgicaux, détecté par le détecteur de rayonnement perturbateur peut être évalué par le calculateur de manière que la division en stades d'EEG pratique dans le cadre d'une anesthésie ou de soins intensifs soit corrigée ou supprimée.

FIG. 1

FIG.2

EP 0 856 181 B1

FIG. 3a

FIG. 3b